# EUROPÄISCHE PATENTSCHRIFT

(11) **EP 0 380 751 B1**
(45) Veröffentlichungstag und Bekanntmachung des Hinweises auf die Patenterteilung: **30.11.1994**
(21) Anmeldenummer: 89117833.7
(22) Anmeldetag: 27.09.1989
(51) Int. Cl.: G01N 33/28

(54) **Vorrichtung zur Feststellung des Alkoholgehaltes und/oder des Heizwertes von Kraftstoffen**
Apparatus for the determination of the alcohol content and/or the calorific value of fuels
Appareil de détermination de la teneur en alcool et/ou la valeur calorifique de carburants

(30) Priorität: 08.12.1988 DE 3841318
(43) Veröffentlichungstag der Anmeldung: 08.08.1990
(73) Patentinhaber: FEV Motorentechnik GmbH & Co. KG, D-52078 Aachen (DE)
(72) Erfinder: Schmitz, Günter, Dr.-Ing., D-5100 Aachen (DE)
(74) Vertreter: Langmaack, Jürgen, Dipl.-Ing.

(56) Entgegenhaltungen:
- PATENT ABSTRACTS OF JAPAN, Band 6, Nr. 133 (R-129)[1011], 20. Juli 1982#

## Beschreibung

Die Erfindung bezieht sich auf eine Vorrichtung zur Feststellung des Alkoholgehaltes und/oder des Heizwertes von Kraftstoffen durch Messung von Kenngrößen des Kraftstoffs in einer Meßzelle und einer elektronischen Meßschaltung.

In Anbetracht der langfristig nur in begrenztem Umfang verfügbaren Reserven an fossiler Energie, insbesondere der aus Rohöl gewonnenen Kraftstoffe, und in Anbetracht der steigenden Anforderungen an den Umweltschutz, wird diesen Kraftstoffen in zunehmendem Maße Methyl- oder Äthylalkohol zugemischt. Dabei soll ein beliebiges Tanken sowohl von Reinkraftstoffen als auch von Mischkraftstoffen möglich sein. Bei höheren Alkoholanteilen ist dabei die Kenntnis des Mischungsverhältnisses erforderlich, um eine optimale Arbeitsweise der Brennkraftmaschine zu erreichen und dabei insbesondere eine genaue, den Betriebsverhältnissen angepaßte Kraftstoffzumessung zu ermöglichen. Besondere Probleme bereitet dabei die laufende Feststellung des Alkoholgehaltes des der Brennkraftmaschine im Betrieb laufend zugeführten Kraftstoffs bei Fahrzeugmotoren, bei denen durch das beliebige Tanken der Kraftstoffarten jede mögliche Mischung erreicht werden kann.

In der prioritätsälteren, nach veröffent lichten deutschen Patentanmeldung DE-A-3 841 264 ist eine Vorrichtung der eingangs bezeichneten Art beschrieben, bei der in einer Meßzelle Kenngrößen des Kraftstoffs gemessen und in einer außerhalb der Meßzelle angeordneten Schaltung gemessen bzw. verarbeitet werden. Diese Schaltung ist ihrerseits mit einem Einspritzrechner verbunden, dessen Ergebnisse zur Steuerung einer Kraftstoffzumeßeinrichtung verwertet werden.

Bei Anordnungen dieser Art bestehen insbesondere in Kraftfahrzeugen in der Umgebung, in der die Meßzelle eingesetzt ist, verhältnismäßig rauhe Betriebsbedingungen. Insbesondere kann bei Unterbringung im Motorraum die Temperatur auf für die elektronischen Schaltungen unverträgliche Temperaturen ansteigen, und dies ist vor allem auch kurz nach dem Abschalten des Motors der Fall; d s ist beispielsweise bei der Meßzelle gemäß Patent Abstracts of Japan, Vol.6, No. 133 (P-129)(1011), 20.07.82 der Fall da die Schaltung nur gegen mechanische Einwirzung geschnitzt ist.

Auch treten innerhalb des Motorraumes starke elektromagnetische Störfelder auf, die u.a. durch die Zündanlage des Motors erzeugt werden und die ordnungsgemäße Funktion der elektronischen Schaltungen beeinträchtigen oder gefährden können.

Der Erfindung liegt die Aufgabe zugrunde, eine Vorrichtung zur Feststellung des Alkoholgehaltes und/oder des Heizwertes von Kraftstoffen gemäß dem Oberbegriff des Anspruchs 1 zu schaffen, bei der die Meßschaltung gegen äußere Einflüsse, insbesondere thermischer und elektromagnetischer Art, besser geschützt ist und zugleich auch hinsichtlich der Baugröße und der Fertigungskosten Vorteile erreichbar sind.

Gemäß der Erfindung wird diese Aufgabe mit den im Anspruch 1 ausgegebenen Mitteln gelöst.

Eine solche Maßnahme scheint wegen des in der Meßzelle vorhandenen bzw. strömenden Kraftstoffes zunächst wenig vorteilhaft und konstruktiv schwer zu realisieren sein, jedoch hat es sich gezeigt, daß die vollständige oder teilweise Unterbringung der elektronischen Meßschaltung in der Meßzelle wesentliche Vorteile insbesondere hinsichtlich der Störunempfindlichkeit, der Temperaturunabhängigkeit, der Baugröße und sogar auch der Fertigungskosten ermöglicht.

Durch die Unterbringung der elektronischen Meßschaltung in der Meßzelle kann in vorteilhafter Weise eine Kühlung durch den Kraftstoff erfolgen, so daß die elektronische Schaltung für einen kleineren Temperaturbereich ausgelegt werden kann und Fehlfunktion, Beschädigung oder Zerstörung nicht zu befürchten ist. Auch ist die elektronische Schaltung bereits durch die Wandung der Meßzelle besser gegen elektromagnetische Störeinflüsse geschützt, und dieser Schutz kann durch eine entsprechende Ausbildung des Gehäuses der elektronischen Schaltung zusätzlich verbessert werden.

Ein weiterer Vorteil ist, daß die elektronische Schaltung durch die Unterbringung innerhalb der Meßzelle optimal gegen mechanische Einwirkungen von außen geschützt ist, da beispielsweise auch im rauhen Werkstattbetrieb Beschädigungen verursacht werden könnten.

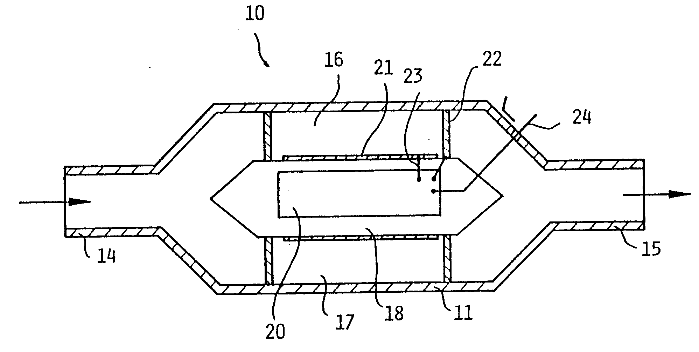

Ein Ausführungsbeispiel der Erfindung wird nachfolgend anhand der Zeichnung näher beschrieben, die vereinfacht und im wesentlichen schematisch einen Längsschnitt durch eine Meßzelle darstellt.

In die Meßzelle 10 mit Wandung 11 gelangt der Kraftstoff über Zufluß 14, und er verläßt die Meßzelle über Abfluß 15. Der Kraftstoffstrom teilt sich in der flächenhaften Darstellung auf in Strömungswege 16 und 17, die durch einen Mittelzylinder 18 gebildet werden. Wenn der Mittelzylinder 18 und die Wandung 11 ganz oder teilweise elektrisch leitend sind, können diese Wandungen bzw. Wandungsteile der Meßzelle 10 die Elektroden des frequenzbestimmenden Kondensators einer Meß- bzw. Auswerteschaltung darstellen. Innerhalb der wenigstens teilweise leitfähigen Wandung der Meßzelle 10, die die erste Elektrode darstellt, ist dann der wenigstens teilweise leitfähige Mittelzylinder 18 als Strömungskörper die zweite Elektrode.

Der Mittelzylinder 18 bildet zusammen mit dem Außenmantel 19 einen Meßkondensator, der das Meßvolumen umschließt.

Im Inneren des Mittelzylinders 18 befindet sich eine elektronische Schaltung 20, die die für den Alkoholgehalt und/oder den Heizwert des Kraftstoffs repräsentativen Meßergebnisse mißt und ggf. auswertet. Die Schaltung 20 ist über Verbindung 23 mit einer Elektrode 21 verbunden, die zu dem Zylinder 18 wandparallel gestaltet oder aber auch als Teil des Zylindergehäuses ausgebildet sein kann. Die Verbindung zur Masse, die gemäß einem vorteilhaften Merkmal zugleich die Verbindung zur durch die Wandung 11 gebildeten Außenelektrode darstellt, wird über eine gleichzeitig als Halterung wirkende Zentrierscheibe 22 hergestellt. Die Signalausgänge der elektronischen Schaltung 20 können über eine Leitung 24 nach außen geführt werden, und sie können anschließend an einen Einspritzrechner weitergegeben werden, der eine Kraftstoffzumeßeinrichtung steuert. Diese Art der Kontaktierung bietet erhebliche fertigungstechnische Vorteile, insbesondere für die Serienfertigung.

Die Erfindung ist nicht auf das dargestellte und beschriebene Ausführungsbeispiel beschränkt. So kann auch bei optisch arbeitenden Sensoren eine Integration der Meßelektronik in den teilweise oder ganz von Kraftstoff umschlossenen oder berührten Teil durchgeführt werden.

Weitere Vorteile ergeben sich, wenn die in der Meßelektronik erzeugten Signale auf nur einer Leitung zusammengefaßt werden und ggf. auch zusammen mit der Spannungsversorgung über nur eine Leitung geführt werden. Dies ist beispielsweise durch eine schaltungstechnische Trennung zwischen Gleich- und Wechselspannungsanteilen möglich.

Weiterhin ist die Erfindung besonders vorteilhaft, wenn mehr als ein Meßwert von der Meßelektronik aufgenommen werden soll. So ist z.B. bei einer getrennten Messung von Leitwert und Kapazität über zwei separate Elektroden der Fertigungsaufwand bei einer Anordnung der Meßelektronik außerhalb des Gehäuses außerordentlich hoch. Durch die Anordnung der Elektronik innerhalb der Meßzelle wird jedoch die fertigungstechnisch aufwendige Kontaktierung umgangen.

Weitere Vorteile können dadurch erreicht werden, daß nicht nur die elektronische Meßschaltung, sondern auch die Schaltung zur Auswertung der von der Meßschaltung gelieferten Signale ebenfalls in der Meßzelle angeordnet ist.

## Patentansprüche

1. Vorrichtung zur Feststellung des Alkoholgehaltes und/oder des Heizwertes von Kraftstoffen durch Messung von Kenngrößen des Kraftstoffs im Durchfluß in einer als Meßkondensator ausgebildeten Meßzelle (10), mit einem Kraftstoffzufluß (14) und einem Kraftstoffabfluß (15), deren Außenwandung (11) eine erste Meßelektrode bildet, der im Innenraum der Meßzelle (10) eine zweite Meßelektrode (21) zugeordnet ist, wobei beide Meßelektroden (11, 21) mit einer elektronischen Schaltungsanordnung (20) verbunden sind, **dadurch gekennzeichnet**, daß die Schaltungsanordnung (20) in der Meßzelle (10) in einem Gehäuse (18) angeordnet ist, das zumindest teilweise von dem vom zu messenden Kraftstoff durchströmten Teil (16, 17) der Meßzelle (10) umschlossen ist.

2. Vorrichtung nach Anspruch 1, dadurch gekennzeichnet, daß die Wandungen des die Schaltungsanordnung (20) umfassenden Gehäuses (18) als Abschirmung gegenüber mechanischen und/oder elektromagnetischen Störeinflüssen ausgebildet sind.

3. Vorrichtung nach Anspruch 1 oder 2, dadurch gekennzeichnet, daß die zweite Meßelektrode (21) an der Außenseite des Gehäuses (18) angebracht ist.

4. Vorrichtung nach Anspruch 3, dadurch gekennzeichnet, daß die zweite Meßelektrode (21) wandparallel an der Außenseite des Gehäuses (18) angeordnet ist.

5. Vorrichtung nach Anspruch 3, dadurch gekennzeichnet, daß die zweite Meßelektrode (21) durch einen Teil der Wandung des Gehäuses (18) gebildet wird.

6. Vorrichtung nach einem der Ansprüche 1 bis 5, dadurch gekennzeichnet, daß das Gehäuse (18) über wenigstens eine mit Durchgangsöffnung versehene Zentrierscheibe (20) mit der Innenwandung der Meßzelle (10) verbunden ist.

## Claims

1. An apparatus for determining the alcohol content and/or the calorific value of fuels by measuring characteristics of the fuel in a flow through a measuring cell (10) designed as a measuring capacitor, with a fuel inflow (14) and a fuel outflow (15), the outer walls (11) of which form a first measuring electrode, with which a second measuring electrode (21) is associated in the interior of the measuring cell (10), both measuring electrodes (11, 21) being connected to an electronic circuit arrangement (20), characterised in that the circuit arrangement (20) in the measuring cell (10) is located in a housing (18) which is at least partly surrounded by the part (16, 17) of the measuring cell (10) through which the fuel to be measured flows.

2. An apparatus according to Claim 1, characterised in that the walls of the housing (18) surrounding the circuit arrangement (20) are constructed as a shield against mechanical and/or electromagnetic perturbing effects.

3. An apparatus according to Claim 1 or 2, characterised in that the second measuring electrode (21) is attached to the outside of the housing (18).

4. An apparatus according to Claim 3, characterised in that the second measuring electrode (21) is arranged parallel to the walls of the outside of the housing (18).

5. An apparatus according to Claim 3, characterised in that the second measuring electrode (21) is formed by a part of the walls of the housing (18).

6. An apparatus according to one of Claims 1 to 5, characterised in that the housing (18) is connected to the inner walls of the measuring cell (10) via at least one centring disc (20) provided with a passage opening.

## Revendications

1. Appareil pour déterminer la teneur en alcool et/ou le pouvoir calorifique de carburants par mesure de caractéristiques du carburant dans son écoulement dans une cellule de mesure (10̸) conçue sous forme de condensateur de mesure, comportant une arrivée du carburant (14) et une sortie du carburant (15), appareil dont la paroi extérieure (11) constitue une première électrode de mesure à laquelle correspond, dans l'espace intérieur de la cellule de mesure (10̸), une seconde électrode de mesure (21), les deux électrodes de mesure (11, 21) étant reliées à un circuit électronique (20̸), appareil caractérisé par le fait que le circuit (20̸) est disposé dans la cellule de mesure (10̸), dans une enceinte (18) qui est, au moins partiellement, enclose par la partie (16, 17) de la cellule de mesure (10̸) à travers laquelle s'écoule le carburant à mesurer.

2. Appareil selon la revendication 1, caractérisé par le fait que les parois de l'enceinte (18) qui enclosent le circuit (20̸) sont conçues en tant qu'écran à l'égard des influences mécaniques et/ou des influences électromagnétiques parasites.

3. Appareil selon la revendication 1 ou 2, caractérisé par le fait que la seconde électrode de mesure (21) est rapportée sur la face extérieure de l'enceinte (18).

4. Appareil selon la revendication 3, caractérisé par le fait que la seconde électrode (21) est disposée contre la face extérieure du boitier (8), parallèlement à sa paroi.

5. Appareil selon la revendication 3, caractérisé par le fait que la seconde électrode de mesure (21) est formée par une partie de la paroi de l'enceinte (18).

6. Appareil selon l'une des revendications 1 à 5, caractérisé par le fait que l'enceinte (18) est reliée à la paroi intérieure de la cellule de mesure (10̸) par au moins une plaque de centrage (20̸) munie d'une ouverture de passage.
